# EUROPEAN PATENT APPLICATION

(11) **EP 1 082 969 A1**
(43) Date of publication of application: **14.03.2001**
(21) Application number: 99830555.1
(22) Date of filing: 07.09.1999
(51) Int. Cl.: A61L 9/12

(54) **Container for release of volatile essences**

(71) Applicant: General FIX S.R.L., 20090 Opera (Milano) (IT)
(72) Inventor: Azzolin, Itala, 20085 Locate Triulzi (Milano) (IT)
(74) Representative: Petruzziello, Aldo

(57) **Abstract**

A container for release of volatile essences comprising a cup (2) of vapour-impermeable material surrounded by a flat frame (4) along which is sealed a multilayer cover sheet (3) comprising, starting from the outside, a layer of impermeable material (5), a layer of peelable material (12) and a layer of vapour-permeable material (13), said multilayer cover sheet (3) not being heat sealed to the frame (4) of the cup in at least one part of the end flap (15) suitable to be gripped by a user, a strip (11) being provided in which said permeable material (13) is removed, comprised between the end flap (15) and the part of multilayer cover sheet (3) heat sealed to the frame (4), so that when the user, by gripping the end flap (15), pulls the multilayer cover sheet (3), he causes removal of the peelable layer (12) whilst the permeable layer (13) remains fixed to the frame (4) of the cup.

## Description

The present invention refers to a container for substances that volatilise, in liquid, solid or gel form, such as fragrances, deodorants and the like. Said container proves particularly suitable for use in contact with heat sources such as inside dishwashers or on burners for dispersion of deodorant essences into the surrounding atmosphere.

Volatile substances, such as perfumed essences, are generally packaged in bottles, plastic containers, or impregnated cards and the like.

For reasons of size, economy of manufacture and practicality of use for the consumer, small containers with osmotic membranes that are permeable or semipermeable to vapour, capable of slow release into the outside atmosphere of the vapour given off by the volatile essence have become widely available on the market.

In this type of container, the permeable membrame is normally coated with a peelable protection that prevents escape of the volatile essence before use.

Commercially known are containers formed by cups made of an impermeable plastic material that acts as a barrier against escape of the vapours, which are closed by means of an osmotic membrane covered by an aluminium sheet that prevents gases from diffusing through the osmotic membrane towards the outside.

The aluminium sheet is bonded to the osmotic membrane which in turn is bonded to the cup of plastic material by sealing or heat sealing. Clearly a part of the end flap of the aluminium sheet must not be bonded to the osmotic membrane, since it must act as a peeling lip for the user. In fact, when the container has to be opened, the user grasps said end of the aluminium sheet and, by pulling, detaches the aluminium sheet from the osmotic membrane thus allowing release of the vapours of volatile substance.

There are various known methods for having part of the end flap of the aluminium sheet not bonded to the osmotic membrane. According to a widespread method, a cut is made in the plastic material of the container while it is hot, down to the aluminium sheet. In this manner, by removing a part of the edge of the container, the end flap of the aluminium sheet where this part has been removed remains free and can therefore be gripped and pulled by the user.

For the same purpose, in some known containers an edge part of the osmotic membrane is treated in a particular way so as not to be heat sealed with the aluminium covering sheet; in this treated area the aluminium sheet remains separate from the osmotic membrane and can therefore be gripped and pulled by the user.

These types of containers according to the prior art have some drawbacks.

The cuts in the plastic of the container and the treatment to prevent heat sealing on the osmotic membrane give rise to problems during industrial production of the container and the resulting container presents difficulties for the removal of the aluminium sheet by the user.

Furthermore, cutting of the plastic of the container while hot cannot be employed for containers destined to be used in hot environments, such as inside dishwashers, washing machines or burners for effusion of deodorants. In fact, plastic materials that withstand high temperatures and are therefore difficult to cut when hot are employed for these uses.

The object of the invention is to eliminate these drawbacks by providing a container for release of volatile essences that is practical, versatile, simple to make and can also be used in high-temperature environments.

This object is achieved in accordance with the invention with the container according to independent claim 1 and the method according to independent claim 7.

Preferred embodiments of the invention are apparent from the dependent claims.

For production of the container for release of volatile essences according to the invention, cups are made by heat moulding of rigid plastic material. Said cups are closed by means of a suitable covering material.

Two rolls are used for production of the covering material for the cups: a roll of sheet aluminium and a roll of a laminate or bonded material consisting of a sheet of multilayer material comprising a layer formed by a vapour-permeable membrane, bonded to a layer of peelable plastic material.

The sheet of aluminium and the sheet of laminate material, each coming from its respective roll, are bonded together by means of heat sealing only along their parallel side edges.

A strip of permeable membrane of the laminate is then trimmed respectively in proximity to one of the heat sealing lines.

Lastly the cover sheet thus obtained is bonded to the heat-moulded rigid plastic cup. This bonding is obtained by means of heat sealing along the outer edges of the cup, stopping said heat sealing in proximity to the removed part of permeable membrane.

As a result the completed container has an end flap of aluminium and laminate material not heat sealed to the cup and therefore suitable for gripping by the user.

When the container must be used, the user grips said end flap and pulls; since the permeable membrane is heat sealed to the outer frame of the cup, it remains fixed to the cup, whereas the aluminium is removed with the peelable layer of material beneath.

It is obvious that this system is extremely simple and practical for the user, is suitable for industrial manufacture, and does not need cuts in the rigid plastic material of the cup or any particular treatment of the osmotic membrane which would be complicated and impractical.

Further characteristics of the invention will be made clearer by the detailed description that follows, referring to a purely exemplary and therefore non limiting embodiment thereof, illustrated in the appended drawings, in which:
Figure 1 is a perspective view of a container for volatile essences according to the invention;
Figure 2 is a view, like Figure 1, of the container with an end flap of the covering sheet raised;
Figure 3 is a diagrammatic view of a manufacturing system for the cover sheet of the container according to the invention;
Figure 4 is a plan view of a multilayer sheet obtained from the system in Figure 3;
Figure 5 is a plan view of the multilayer sheet in Figure 4 at the end of the manufacturing process;
Figure 6 is a cross section of the multilayer sheet, along the plane VI-VI of Figure 5;
Figure 7 is a cross section of a part of the edge of the container along the line VII-VII of Figure 1.

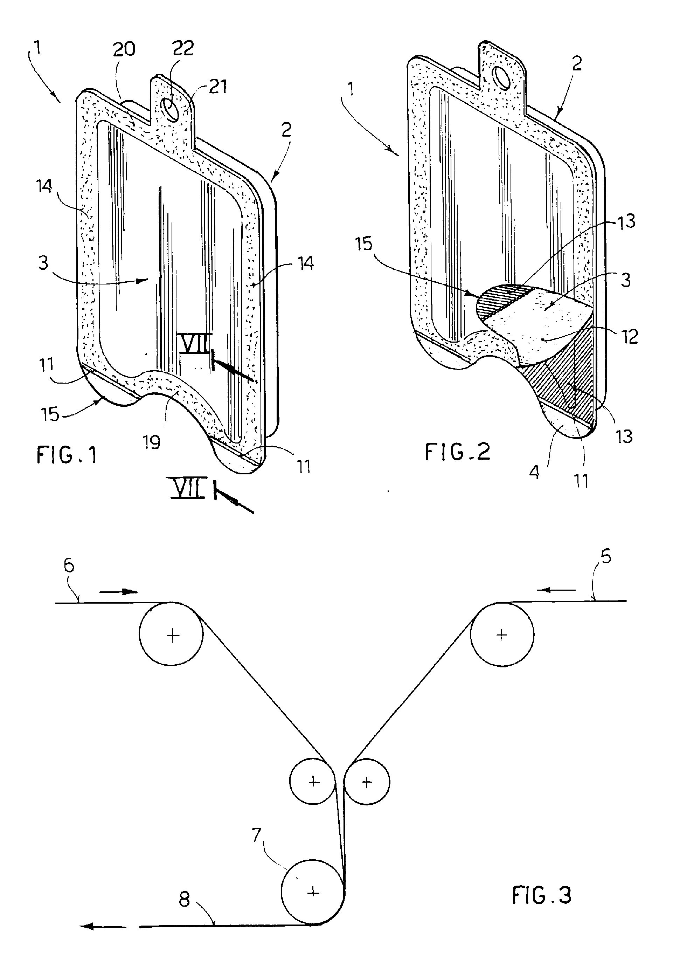

Figure 1 shows a container according to the invention denoted as a whole by reference numeral 1. The container 1 comprises a substantially rectangular shaped cup 2 closed by means of a cover sheet 3. The cup 2 is made by heat moulding using rigid vapour-impermeable plastic material. The cup 2 has a flattened outer frame 4, substantially rectangular in shape in the example shown, on which the cover sheet 3 is fixed by heat sealing.

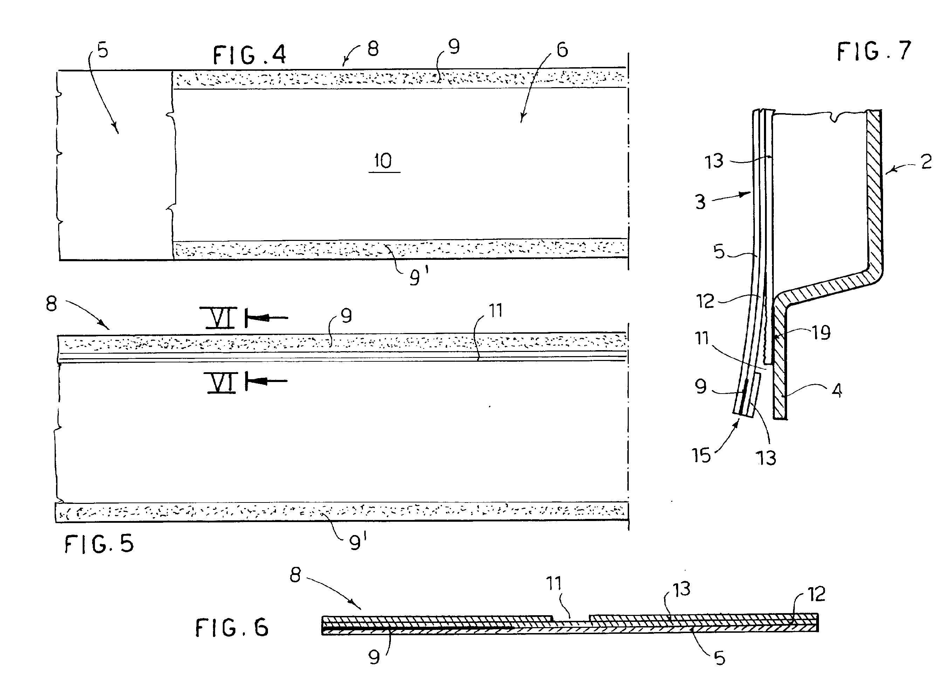

The manufacturing method of the cover sheet 3 and its structure are explained with the aid of Figure 3. A sheet 5 of impermeable material, such as aluminium for example, is unrolled from the first reel and a sheet of laminate material 6 is unrolled from the second reel. The sheet of laminate 6 is made from a multilayer material comprising a layer 13 formed of vapour-permeable material and a layer 12 of peelable material (see Figures 6 and 7). A thin layer of suitably treated PET, for example, to be considered per se known, , which is able to allow the vapours given off by volatile substances to permeate very slowly, can be used as the vapour-permeable material.

The sheet of aluminium 5 and the sheet of laminate material 6, by means of respective drive rollers, are directed toward a bonding station 7 in which they are joined together by heat sealing, so as to obtain a multilayer sheet 8 leaving the bonding station 7, consisting of a layer of aluminium, a layer of peelable material and a layer of vapour-permeable material.

Bonding of the aluminium sheet 5 and the sheet of laminate material 6, as shown in Figure 4, takes place by means of heat sealing along parallel strips 9, 9' situated along the side edges of the sheet 8. In the central region 10 of the multilayer sheet 8, on the other hand, the sheet of laminate material 6 is not heat sealed to the aluminium sheet.

After this bonding stage a cutting operation is performed, in which, as shown in Figure 5, a strip 11 of permeable material is removed by drawing from the multilayer sheet 8. This removal of the strip 11 of permeable material takes place along a line close to and parallel to one of the two heat-sealed strips, strip 9 with reference to Figure 5.

Figure 6 shows a view in cross section of the multilayer sheet 8, in which the layer of peelable material and the layer of permeable material have been indicated respectively with reference numerals 12 and 13. Starting from the bottom, the layer of aluminium 5, the layer of peelable material 12 heat sealed to the layer of aluminium 5 along the heat sealing line 9 and the layer of permeable material 13 bonded to the layer of peelable material 12 can be seen. The layer of permeable material 13 is interrupted along the strip 11 obtained by removal of the permeable material and disposed near the heat-sealing strip 9.

Once the multilayer sheet 8 has been completed, it can be applied to the cup 2 with the layer of permeable material 13 in contact with the frame 4 of the cup 2 and the edges of the multilayer sheet 8 can thus be cut so as to obtain the cover sheet 3 for the cup 2

As shown in Figure 1, the cover sheet 3 is heat sealed to the frame 4 of the cup 2 along two strips 14 substantially at right angles to the strip 11 of removed permeable material of the cover sheet 3, along a strip 19 that joins the two strips 14 in proximity to the strip 11 of removed permeable material and along an opposite strip 20, where a central region is left open for filling. The heat sealing is interrupted in proximity to the strip 11 of removed material so as to obtain end flaps 15 of the cover sheet 3 that are not heat sealed to the frame 4 of the cup 2.

As shown in Figure 7, the end flap 15 of the cover sheet 3 is detached from the frame 4 of the cup 2, therefore it can easily be grasped by the user.

At this point an injection nozzle is inserted between the cover sheet 3 and the cup 2, on the opposite side to the strip of heat-sealing 19, in the area previously left open, and the volatile material is then injected into the cup 3. Afterwards the injection nozzle is removed and the central area of the strip 20 is also heat sealed between the cover sheet 3 and the frame 4 of the cup.

The container 1 can have a plate 21 with a hole 22 able to accommodate means for hanging or hooking the container 1.

For opening of the container 1, as shown in Figure 2, the user grips the end flap 15 and pulls. The layer of permeable material 13 is heat sealed to the frame 4 of the cup, along the heat sealing strip 19, in the part after the removed strip 11. Following pulling of the end flap 15, therefore, the layer of impermeable material 13 remains fixed to the frame 4, whereas the cover sheet 3 detaches along the peelable layer 12.

Consequently, after opening of the container 1, the cover sheet 3 formed by the layer of aluminium 5 and the layer of peelable material 12 is removed except for the part of the end flap 15 that comprises the layer of aluminium 5, the layer of peelable material 12 and the layer of permeable material 13.

The cup 2, on the other hand, remains covered only by the layer of permeable material 13 which remains perfectly heat sealed to its frame 4. In this manner the volatile material inside the cup 2 can diffuse to the outside through the layer of permeable material 13.

## Claims

1. A container for release of volatile essences comprising a cup (2) of vapour-impermeable material surrounded by a flat frame (4) along which is heat sealed a multilayer cover (3) comprising, from the outside, a layer of impermeable material (5), a layer of peelable material (12) and a layer of vapour-permeable material (13) with an end pull flap (15) suitable to be gripped by a user, to allow removal of said layer of impermeable material (5), characterized in that said multilayer cover sheet (3) has a strip (11) without said permeable material (13), said strip (11) being comprised between said end flap (15) which is not heat sealed to said frame (4) and the part of multilayer cover sheet (3) heat sealed to the frame (4), so that when the user, gripping the end flap (15), pulls the multilayer cover sheet (3), he causes removal of the peelable layer (12), whilst the permeable layer (13) remains fixed to the frame (4) of the cup.

2. A container according to claim 1, characterized in that said layer of permeable material (13) of the cover sheet (3) is PET based.

3. A container according to claim 1 or 2, characterized in that said layer of impermeable material (5) of the cover sheet (3) is made of aluminium.

4. A container according to any one of the preceding claims, characterized in that said cover sheet (3) is made through heat sealing to join said layer of impermeable material (5) and a sheet of laminate material (6) comprising said layer of peelable material (12) and said layer of permeable material (13).

5. A container according to claim 4, characterized in that said heat sealing to join the sheet of impermeable material (5) and the sheet of laminate material (6) is done along parallel strips (9, 9'), situated in proximity to the side edges of the cover sheet (3).

6. A container according to any one of the preceding claims, characterized in that it has a hole (22) able to accommodate means to support the container.

7. A method for manufacturing a container for release of volatile essences comprising a cup (2) surrounded by a flat frame (4) on which is applied a multilayer cover sheet (3) formed, from the outside inward, by a layer of impermeable material (5), a layer of peelable material (12) and a layer of permeable material (13), the method comprising the following steps:
- manufacture of the multilayer cover sheet (3) starting from a sheet of impermeable material (5) that is joined, by heat sealing, to a sheet of laminate comprising said layer of peelable material (12) bonded to said layer of permeable material (13);
- removal of a strip (11) of the layer of permeable material (13) from said multilayer cover sheet (3);
- joining of the multilayer cover sheet (3) to said frame (4) of the cup (2), by means of heat sealing, excluding an end flap (15) of the cover sheet (3) beyond the strip (11) of removed permeable material.

8. A method according to claim 7, characterized in that said sheet of impermeable material (5) is joined to said sheet of laminate by heat welding along side strips (9, 9').

9. A method according to claim 8, characterized in that said strip (11), along which said permeable material (13) is removed, is situated parallel to and inside one of said lateral heat sealing strips (9, 9').
